# EUROPEAN PATENT APPLICATION

(11) **EP 1 369 408 A1**
(43) Date of publication of application: **10.12.2003**
(21) Application number: 02702875.2
(22) Date of filing: 11.03.2002
(51) Int. Cl.: C07C 51/377, C07C 62/24, C07C 67/327, C07C 69/757

(54) **PROCESS FOR PRODUCING BICYCLOCARBOXYLIC ACID DERIVATIVE**

(30) Priority: 14.03.2001 JP 2001071743
(71) Applicant: TAISHO PHARMACEUTICAL CO., LTD, Tokyo 170-8633 (JP)
(72) Inventor: NAKAZATO, Atsuro, Toshima-ku, Tokyo 170-8633 (JP); KUMAGAI, Toshihito, Toshima-ku, Tokyo 170-8633 (JP); SAKAGAMI, Kazunari, Toshima-ku, Tokyo 170-8633 (JP)
(74) Representative: HOFFMANN EITLE
(86) International application number: JP0202254
(87) International publication number: WO02072525

(57) **Abstract**

A process for producing a 2-oxobicyclo[3.1.0]hex-3-ene-6-carboxylic acid derivative represented by a formula: 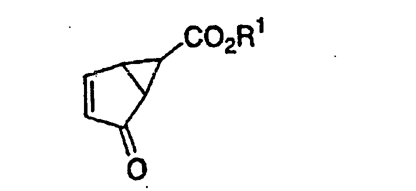 (wherein R¹ represents a hydrogen atom, a C₁₋₆ alkyl group, a C₃₋₆ cycloalkyl group, a C₃₋₆ cycloalkyl C₁₋₆ alkyl group, a C₁₋₆ alkyl group substituted with a substituted or unsubstituted phenyl group, a C₁₋₆ alkoxy C₁₋₆ alkyl group, a C₁₋₆ hydroxyalkyl group, a C₁₋₆ alkylthio C₁₋₆ alkyl group, a C₁₋₆ mercaptoalkyl group, or a substituted or unsubstituted phenyl group)
characterized by reacting a 4-hydroxy-2-oxobicyclo[3.1.0]hexane-6-carboxylic acid derivative represented by a formula: 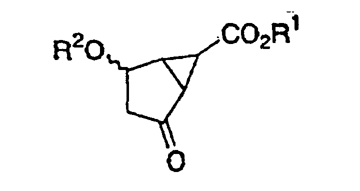 (wherein R¹ has the same meaning as described above, and R² represents a hydrogen atom or a protective group of a hydroxyl group)
in the presence of an acid or a base.

A process for producing the compounds which are useful for efficient syntheses of 2-amino-3-fluorobicyclo[3.1.0]hexane-2,6-dicarboxylic acids is provided.

## Description

### Technical Field

The present invention relates to a process for producing a 2-oxobicyclo[3.1.0]hex-3-ene-6-carboxylic acid derivative.

### Background Art

The metabotropic glutamate receptors, which are one type of glutamate receptor, are classified pharmacologically into three groups. Of these, group 2 (mGluR2/mGluR3) bind with adenylcyclase, and inhibit the accumulation of the Forskolin stimulation of cyclic adenosine monophosphate (cAMP) *(Trends Pharmacol. Sci.,* 14, 13 (1993)), and for this reason, it is suggested that the compounds acting on group 2 metabotropic glutamate receptors have treatment effects and prevention effects on psychiatric disorders such as schizophrenia, anxiety and its associated diseases, depression, bipolar disorder, and epilepsy; and on neurological diseases such as drug dependence, cognitive disorders, Alzheimer's disease, Huntington's chorea, Parkinson's disease, dyskinesia associated with muscular stiffness, cerebral ischemia, cerebral failure, myelopathy, and head trauma.

In addition, International Publication No. WO 99/38839 describes a 2-amino-3-fluorobicyclo[3.1.0]hexane-2,6-dicarboxylic acid (3) as a compound acting on group 2 metabotropic glutamate receptors. Furthermore, as a process for producing the same, International Publication No. WO 00/37410 proposes a process for producing a 2-amino-3-fluorobicyclo[3.1.0]hexane-2,6-dicarboxylic acid (3) as shown in Reaction Scheme 1 described below (wherein R¹ has the same meaning as described in the definition of the present Specification).

In addition, Compound (1) and a process for producing the same are described in International Publication No. WO 00/58258, as shown in Reaction Scheme 2 described below (wherein R¹ and R² have the same meanings as described in the definition of the present Specification). However, there are no descriptions regarding a process for producing Compound (2) (including Compound (2')) from Compound (1).

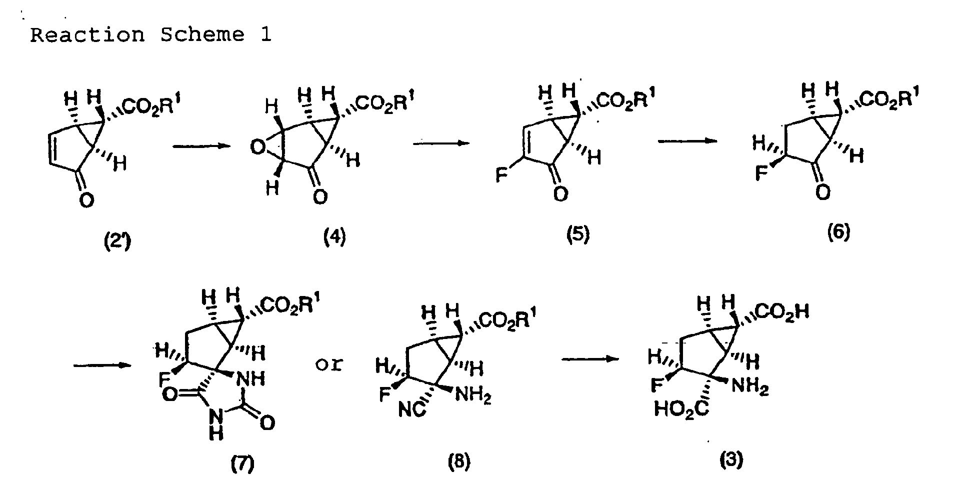

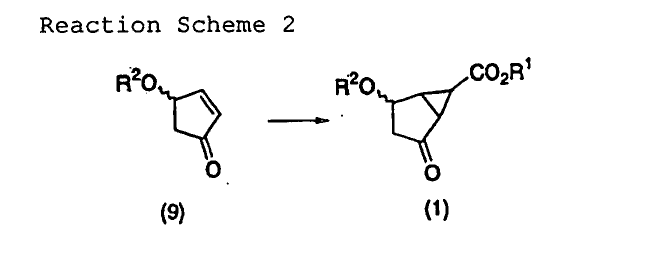

An objective of the present invention is to provide a process for producing a 2-oxobicyclo[3.1.0]hex-3-ene-6-carboxylic acid derivative (2) which is a synthesis intermediate useful for an efficient synthesis of 2-amino-3-fluorobicyclo[3.1.0]hexane-2,6-dicarboxylic acid acting on group 2 metabotropic glutamate receptors, which have treatment effects and prevention effects on psychiatric disorders such as schizophrenia, anxiety and its associated diseases, depression, bipolar disorder, and epilepsy; and on neurological diseases such as drug dependence, cognitive disorders, Alzheimer's disease, Huntington's chorea, Parkinson's disease, dyskinesia associated with muscular stiffness, cerebral ischemia, cerebral failure, myelopathy, and head trauma.

### Disclosure of the Invention

As a result of diligent research in order to achieve the objective described above, the present inventors discovered an efficient process for producing a 2-oxobicyclo[3.1.0]hex-3-ene-6-carboxylic acid derivative (2) which is a synthesis intermediate useful for a synthesis of 2-amino-3-fluorobicyclo[3.1.0]hexane-2,6-dicarboxylic acid, and consequently, have completed the present invention.

That is, the present invention relates to a process for producing a 2-oxobicyclo[3.1.0]hex-3-ene-6-carboxylic acid derivative represented by Formula (2): 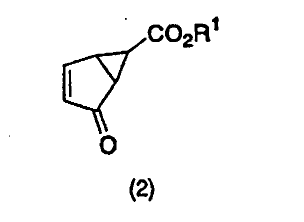 (wherein R¹ represents a hydrogen atom, a C₁₋₆ alkyl group, a C₃₋₆ cycloalkyl group, a C₃₋₆ cycloalkyl C₁₋₆ alkyl group, a C₁₋₆ alkyl group substituted with a substituted or unsubstituted phenyl group, a C₁₋₆ alkoxy C₁₋₆ alkyl group, a C₁₋₆ hydroxyalkyl group, a C₁₋₆ alkylthio C₁₋₆ alkyl group, a C₁₋₆ mercaptoalkyl group, or a substituted or unsubstituted phenyl group)
characterized by reacting a 4-hydroxy-2-oxobicyclo[3.1.0]hexane-6-carboxylic acid derivative represented by Formula (1): 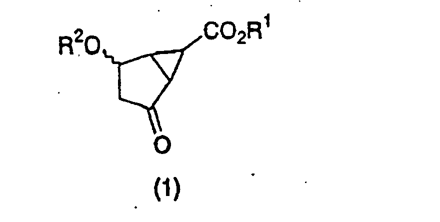 (wherein R¹ has the same meaning as described above, and R² represents a hydrogen atom or a protective group of a hydroxyl group)
in the presence of an acid or a base.

The terms used in the present invention are defined in the following. In the present invention, "Cₙ₋ₘ" means that the group following the "Cₙ₋ₘ" has from n to m carbon atoms.

The C₁₋₆ alkyl group means a straight-chain or branched-chain alkyl group, examples of which include a methyl group, an ethyl group, a propyl group, an isopropyl group, a butyl group, an isobutyl group, a t-butyl group, a pentyl group, an isopentyl group, a 1-ethylpropyl group, a hexyl group, an isohexyl group, a 2-ethylbutyl group, a heptyl group, an isopentyl group, a hexyl group, an isohexyl group, and the like. The C₃₋₆ cycloalkyl group means, for example, a cyclopropyl group, a cyclobutyl group, a cyclopentyl group, a cyclohexyl group, or the like. The C₃₋₆ cycloalkyl C₁₋₆ alkyl group means, for example, a cyclopropylmethyl group, a cyclobutylmethyl group, a cyclopentylmethyl group, a cyclohexylmethyl group, or the like. The substituted or unsubstituted phenyl group means a phenyl group having 1 to 3 substituents on the benzene ring, the substituents being any substituents selected from a hydrogen atom, a halogen atom such as a fluorine atom, a chlorine atom, a bromine atom, or the like, a C₁₋₆ alkyl group, a C₁₋₆ alkanoyl group, a cyano group, a nitro group, a hydroxy group, and a C₁₋₆ alkoxy group such as a methoxy group or the like. The C₁₋₆ alkyl group substituted with a substituted or unsubstituted phenyl group means a straight-chain or branched-chain alkyl group substituted with one or two phenyl groups, examples of which include a benzyl group, a diphenylmethyl group, a 1-phenylethyl group, 2-phenylethyl group, a 4-methoxybenzyl group, and the like. The C₁₋₆ alkoxy C₁₋₆ alkyl group means a straight-chain or branched-chain alkoxyalkyl group, examples of which include a methoxymethyl group, an ethoxymethyl group, a methoxyethyl group, an ethoxyethyl group, a propoxyethyl group, an isopropoxyethyl group, a butoxyethyl group, an isobutoxyethyl group, a pentyloxyethyl group, an isopentyloxyethyl group, and the like. The C₁₋₆ hydroxyalkyl group means, for example, a 2-hydroxyethyl group, a 3-hydroxypropyl group, a 2,3-dihydroxypropyl group, or the like. The C₁₋₆ alkanoyl group means a straight-chain or branched-chain alkanoyl group, examples of which include a formyl group, an acetyl group, a pivaloyl group, and the like. The C₁₋₆ alkylthio C₁₋₆ alkyl group means a straight-chain or branched-chain alkylthioalkyl group, examples of which include a methylthiomethyl group, a 2-methylthioethyl group, and the like. The C₁₋₆ mercaptoalkyl means, for example, a 2-mercaptoethyl group, a 3-mercaptopropyl group, a 2,3-dimercaptopropyl group, or the like.

In each of the groups descried above, at least one hydrogen atom on the group may be substituted with, for example, a non-hydrogen atom or with another group, such as a halogen atom such as a fluorine atom, a chlorine atom, a bromine atom, an iodine atom, or the like; a nitro group; an amino group; a hydroxyl group; a thiol group; a formyl group; a carboxyl group; a cyano group; a carbamoyl group; an alkyl group such as a methyl group, an ethyl group, a propyl group, an isopropyl group, a butyl group, an isobutyl group, a sec-butyl group, a t-butyl group, a pentyl group, an isopentyl group, a neopentyl group, a t-pentyl group, or the like; an aryl group and a heterocyclic group such as a phenyl group, a naphthyl group, a biphenyl group, an anthranyl group, a pyrrolyl group, a pyridyl group, a thienyl group, or the like; an alkoxycarbonyl group such as a methoxycarbonyl group, an ethoxycarbonyl group, or the like; an acyl group such as an acetyl group, a benzoyl group, or the like; an alkoxy group such as a methoxy group, an ethoxy group, a propoxy group, or the like; or an alkylthio group such as a methylthio group, an ethylthio group, a propylthio group, or the like. Therefore, for example, a 2,2,2-trichloroethyl group, a 2,6-dimethylcyclohexan-1-yl group, a 2,4-dimethylpentan-3-yl group, and the like are also included in the scope of R¹. The number of the carbon atoms in these substituents is excluded from the numbers n or m-described above. The protective derivative of a hydroxyl group means a form in which a protection is carried out by a general protective group of a hydroxyl group, examples of which include a silyloxy derivative such as a trimethylsilyloxy group, a t-butyldimethylsilyloxy group, or the like, or an acylated derivative such as an acetoxy group, a trifluoroacetoxy group, or the like.

In the compounds represented by Formula (1), there are four asymmetric carbon atoms; and in the compounds represented by Formula (2), there are three asymmetric carbon atoms. Therefore, the compounds of the present . invention can be present as optically active substances, enantiomers thereof, an enantiomer mixture such as a racemic modification, and a diastereomer mixture. That is, the compounds of the present invention include all the optically active substances of the compounds represented by Formula (1) and Formula (2), enantiomers thereof, an enantiomer mixture such as a racemic modification, and a diastereomer mixture.

The compounds of Formula (2) in the present invention can be produced by the reaction described below.

In the following reaction schemes, R¹ and R² have the same meanings as described above; X¹ represents a chlorine atom, a bromine atom, an iodine atom, or a sulfonyloxy group such as a trifluoromethanesulfonyloxy group, a methanesulfonyloxy group, a p-toluenesulfonyloxy group, or the like; and X² represents a group represented by a formula: R³S(O)ₙ- or R³Se(O)ₙ- (wherein R³ represents a methyl group, an ethyl group, or a phenyl group; and n represents an integer of from 0 to 2). In addition, common protection and deprotection of a hydroxy group employed in the reactions are described in detail in *PROTECTIVE GROUPS IN ORGANIC SYNTHESIS,* written by THEODORA W. GREENE and PETER G.M. WUTS, the description of which is incorporated by reference in the specification of the present specification.

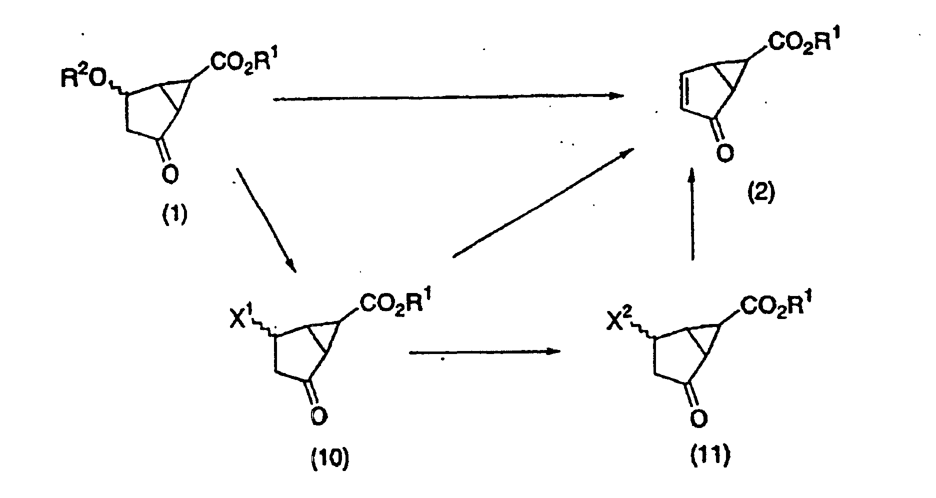

Compound (1) wherein R² does not represent a hydrogen atom can be converted into Compound (2) by reacting the Compound (1), after the protective group of the hydroxyl group of which is deprotected under common deprotective conditions or remains as it is, in the presence of an acid or a base, in an inert solvent, examples of which include a hydrocarbon type solvent such as benzene, toluene, or hexane; a halogen type solvent such as dichloromethane or chloroform; an alcohol such as methanol, ethanol, or isopropyl alcohol; an ether such as tetrahydrofuran, diethyl ether, or 1,2-dimethoxyethane; an amide such as N,N-dimethylformamide or N-methylpyrrolidone; acetonitrile; dimethylsulfoxide; hexamethylphosphoramide; or a mixture of these solvents. Here, the base indicates, for example, an amine such as triethylamine, diisopropylethylamine, pyridine, or 1,8-diazabicyclo[5.4.0]-7-undecene; an inorganic base such as potassium carbonate, sodium carbonate, or sodium hydrogencarbonate; a metal alcholate such as sodium methoxide, sodium ethoxide, or potassium t-butoxide; or the like. The acid indicates, for example, an inorganic acid such as hydrogen chloride, hydrogen bromide, or sulfuric acid; or an organic acid such as p-toluenesulfonic acid, methanesulfonic acid, trifluoroacetic acid, or acetic acid. In addition, Compound (1) wherein R² represents a hydrogen atom can be converted into Compound (2) by dehydrating the Compound (1) in the presence of, for example, phosphoryl chloride-pyridine, thionyl chloride-pyridine, or the like.

In addition, Compound (1) wherein R² represents a hydrogen atom can be converted into Compound (2) by reacting the Compound (1) with a sulfonylation reagent such as anhydrous trifluoromethanesulfonic acid, N-phenylbis(trifluoromethanesulfonimide), methanesulfonyl chloride, or p-toluenesulfonyl chloride, in the presence or absence of a base, examples of which include an amine such as triethylamine, diisopropylethylamine, pyridine, or 4-dimethylaminopyridine; an inorganic base such as potassium carbonate or sodium hydrogencarbonate; or the like, in an inert solvent, examples of which include a hydrocarbon type solvent such as benzene, toluene, or hexane; a halogen type solvent such as dichloromethane or chloroform; an ether such as tetrahydrofuran, diethyl ether, or 1,2-dimethoxyethane; an amide such as N,N-dimethylformamide or N-methylpyrrolidone; acetonitrile; or a mixture of these solvents. Alternatively, Compound (1) wherein R² represents a hydrogen atom can be converted into Compound (2) by reacting the Compound (1) with a halogenation reagent such as thionyl chloride, oxalyl chloride, triphenylphosphine-carbon tetrabromide, N-bromosuccinic imide-triphenylphosphine, or iodine-triphenylphosphine to produce Compound (10), followed by a reaction with an amine such as triethylamine, diisopropylethylamine, pyridine, or 1,8-diazabicyclo[5.4.0]-7-undecene; an inorganic base such as potassium carbonate, sodium hydrogencarbonate, or sodium hydride; or a metal alcholate such as sodium methoxide or potassium t-butoxide, in an inert solvent, examples of which include a hydrocarbon type solvent such as benzene, toluene, or hexane; a halogen type solvent such as dichloromethane or chloroform; an ether such as tetrahydrofuran, diethyl ether, or 1,2-dimethoxyethane; an amide such as N,N-dimethylformamide or N-methylpyrrolidone; acetonitrile; or a mixture of these solvents.

Furthermore, from the Compound (10), the Compound (2) can be produced by converting the Compound (10) into Compound (11) by a reaction with a thiol such as benzene thiol or methane thiol, or a selenol such as benzene selenol, in the presence of a base such as sodium hydride, potassium carbonate, or triethylamine, and subsequently reacting in the presence of a base, or alternatively oxidizing with an oxidant such as m-chloroperbenzoic acid or hydrogen peroxide, followed by a reaction in the presence or absence of a base in an inert solvent. Here, the inert solvent indicates, for example, a hydrocarbon type solvent such as benzene, toluene, or hexane; a halogen type solvent such as dichloromethane or chloroform; an alcohol such as methanol, ethanol, or isopropyl alcohol; an ether such as tetrahydrofuran, diethyl ether, or 1,2-dimethoxyethane; an amide such as N,N-dimethylformamide or N-methylpyrrolidone; acetonitrile; diemthylsulfoxide; hexamethylphosphoramide, or a mixture of these solvents. In addition, the base indicates, for example, an amine such as triethylamine, diisopropylethylamine, pyridine, or 1,8-diazabicyclo[5.4.0]-7-undecene; an inorganic base such as potassium carbonate, sodium carbonate, or sodium hydrogencarbonate; a metal alcholate such as sodium methoxide, sodium ethoxide, or potassium t-butoxide; or the like.

Here, in the case where Compound (2) is a diastereomer mixture, the diastereomers may be separated by a common column chromatography using silica gel or recrystallization. In addition, in the case where Compound (2) is an enantiomer mixture such as a racemic modification, the enantiomers can be optically resolved into (+) and (-) optically active substances by the HPLC method employing chiral carriers such as cellulose carbamate derivatives or amylose carbamate derivatives. Alternatively, they can be optically resolved into (+) and (-) optically active substances by converting the R¹ into a hydrogen atom by means of a common hydrolysis of an ester moiety (a method described in *PROTECTIVE GROUPS IN ORGANIC SYNTHESIS,* written by THEODORA W. GREENE and PETER G.M. WUTS), followed by forming salts with optically active amines such as (+)- or (-)-1-phenylethylamine, (+)-or (-)-phenylglycinol, (+)- or (-)-2-amino-1-butanol, (+)-or (-)-alaninol, brucine, cinchonidine, cinchonine, quinine, quinidine, or dehydroabiethylamine, or by deriving to the amide derivatives with optically active primary or secondary amines.

### Best Modes For Carrying Out the Invention

In the following, representative examples of the present invention are described. It should be understood that the present invention is not limited to these examples.

### Example 1

### Synthesis of ethyl (1SR,5RS,6SR)-2-oxobicyclo[3.1.0]hex-3-ene-6-carboxylate

To a chloroform (10 mL) solution of 1.03 g of a mixture of ethyl (1SR,4RS,5RS,6SR)-4-t-butyldimethylsilyloxy-2-oxobicyclo[3.1.0]hexane-6-carboxylate and ethyl (1SR,4SR,5RS,6SR)-4-t-butyldimethylsilyloxy-2-oxobicyclo[3.1.0] hexane-6-carboxylate, 0.13 mL of boron trifluoride-diethyl ether complex was added at room temperature. The reaction was carried out for 15 hours at room temperature. The reaction mixture was poured into a saturated aqueous solution of sodium hydrogencarbonate-ice, followed by extracting with chloroform three times and extracting with ethyl acetate twice. The organic phases were combined, and were dried over anhydrous sodium sulfate. After the desiccant was filtered off, the filtrate was concentrated under reduced pressure. The obtained residue, in an amount of 0.72 g, and p-toluenesulfonic acid monohydrate, in an amount of 66 mg, were diluted with 6 mL of benzene, followed by refluxing by heating for 1.5 hours. p-toluenesulfonic acid monohydrate, in an amount of 66 mg, was added thereto, followed by further refluxing by heating for one and a half hour. The reaction mixture was cooled to room temperature, and was separated into a saturated aqueous solution of sodium hydrogencarbonate and ethyl acetate. The aqueous phase was extracted with ethyl acetate twice. Subsequently, the organic phases were combined and were dried over anhydrous sodium sulfate. After the desiccant was filtered off, the filtrate was concentrated under reduced pressure. The obtained residue was purified by silica gel column chromatography (Wakogel C-200; hexane/ethyl acetate = 7:1) to yield 0.44 g of ethyl (1SR,5RS,6SR)-2-oxobicyclo[3.1.0]hex-3-ene-6-carboxylate.
m.p. 77 to 78°C.

### Industrial Applicability

According to the present invention, there is provided an efficient process for producing 2-oxobicyclo[3.1.0]hex-3-ene-6-carboxylic acid derivatives (2) (the present compounds are useful as synthesis intermediates of 2-amino-3-fluorobicyclo [3.1.0]hexane-2,6-dicarboxylic acid derivatives (3) which act on group 2 metabotropic glutamate receptors having treatment effects and prevention effects of psychiatric disorders such as schizophrenia, anxiety and its associated diseases, depression, bipolar disorder, and epilepsy; and on neurological diseases such as drug dependence, cognitive disorders, Alzheimer's disease, Huntington's chorea, Parkinson's disease, dyskinesia associated with muscular stiffness, cerebral ischemia, cerebral failure, myelopathy, and head trauma, described in WO 99/38839), and for this reason, 2-amino-3-fluorobicyclo[3.1.0]hexane-2,6-dicarboxylic acid derivatives (3) described in WO 99/38839 can be efficiently produced.

## Claims

1. A process for producing a 2-oxobicyclo[3.1.0]hex-3-ene-6-carboxylic acid derivative represented by a formula: 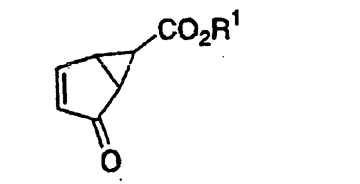 (wherein R¹ represents a hydrogen atom, a C₁₋₆ alkyl group, a C₃₋₆ cycloalkyl group, a C₃₋₆ cycloalkyl C₁₋₆ alkyl group, a C₁₋₆ alkyl group substituted with a substituted or unsubstituted phenyl group, a C₁₋₆ alkoxy C₁₋₆ alkyl group, a C₁₋₆ hydroxyalkyl group, a C₁₋₆ alkylthio C₁₋₆ alkyl group, a C₁₋₆ mercaptoalkyl group, or a substituted or unsubstituted phenyl group)
**characterized by** reacting a 4-hydroxy-2-oxobicyclo[3.1.0]hexane-6-carboxylic acid derivative represented by a formula: 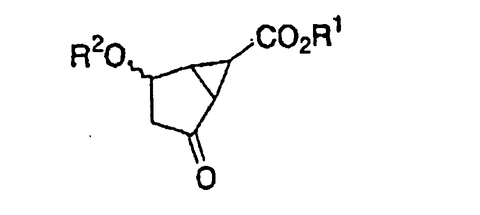 (wherein R¹ has the same meaning as described above, and R² represents a hydrogen atom or a protective group of a hydroxyl group)
in the presence of an acid or a base.

2. The process for producing a 2-oxobicyclo[3.1.0]hex-3-ene-6-carboxylic acid derivative according to Claim 1, wherein R¹ is a hydrogen atom or a C₁₋₆ alkyl group.
